# EUROPEAN PATENT APPLICATION

(11) **EP 0 695 760 A1**
(43) Date of publication of application: **07.02.1996**
(21) Application number: 94202268.2
(22) Date of filing: 05.08.1994
(51) Int. Cl.: C07K 14/47, C07K 16/30, G01N 33/574, C12N 5/28, A61K 49/00, A61K 39/00, A61K 39/395

(54) **Novel tumor marker for lung cancer**

(71) Applicant: F. HOFFMANN-LA ROCHE AG, CH-4002 Basel (CH)
(72) Inventor: Cocciolo, Maria Gabriella, I-20091 Bresso (MI) (IT); Maurer, Andreas, CH-4303 Kaiseraugst (CH); Pizzorno, Beatrice, I-20131 Milano (IT); Grassi, Cinzia, I-20096 Pioltello (MI) (IT)
(74) Representative: Appoloni, Romano

(57) **Abstract**

The invention provides a new tumor marker for lung cancer, which is derived from a lung cancer antigen which is a glycoprotein recognized by a monoclonal antibody selected from the group consisting of monoclonal antibodies P14B4 (DSM ACC2170) and P6F7 (DSM ACC2171), a monoclonal antibody recognizing this lung cancer antigen, a reagent and a test kit for diagnosis of lung cancer, an imaging agent, an immunotoxin and a vaccine, which contain such a monoclonal antibody, as well as methods for diagnosing lung cancer which use such a monoclonal antibody.

## Description

The invention relates to a new tumor marker for lung cancer, which is derived from a lung cancer antigen which is a glycoprotein recognized by a monoclonal antibody selected from the group consisting of monoclonal antibodies P14B4 (DSM ACC2170) and P6F7 (DSM ACC2171).

The invention also relates to a monoclonal antibody recognizing this lung cancer antigen, a reagent and a test kit for diagnosis of lung cancer, an imaging agent, an immunotoxin and a vaccine, which comprise such a monoclonal antibody, as well as methods for diagnosing lung cancer which use such a monoclonal antibody.

Lung cancer is currently one of the most common causes of death in industrial countries. In the United States about 85,000 men and 36,000 women die of lung cancer every year. In Italy the figures are 13,000 and 2,000 for males and females, respectively. The overall annual mortality rate in industrial countries amounts to 28 per 100,000 inhabitants.

About 650,000 lung cancer cases are diagnosed annually worldwide. Lung cancer may be suspected in presence of unspecific symptoms such as cough, dyspnea, hemoptysis, infections and abnormal chest X-rays, but final diagnosis can only be made by pathological examination. Histological differentiation between Small Cell Lung Cancer (SCLC) and Non Small Cell Lung Cancer (NSCLC), as well as staging of NSCLC, are of great importance for the choice of therapy. SCLC is a metastatic disease which must be treated by chemotherapy whereas it is often possible to treat sucessfully by surgery or radiotherapy the early stages of NSCLC for patients without histologically proven metastatic disease. NSCLC constitutes approximately 80% of all lung cancers and includes three different histology groups: squamous cells carcinoma, adenocarcinoma and large cells carcinoma.

Tumor markers are proteins or peptides that are found in the blood of tumor patients at elevated concentrations compared to healthy people. They are useful for staging, monitoring and follow-up of tumor patients. A correlation is often found between the tumor marker level and tumor mass in a given type of cancer.

The change of the tumor marker level, as well as its value compared to the average level found in healthy people, are used for monitoring cancer therapy, a persistent rise or a value above a defined cut off being indicative of recurrent disease. Tumor progression is however sometimes difficult to differentiate from treatment complications such as infections or inflammations, which may also elevate the tumor marker level. In a few cases tumor markers can also be used for screening persons suspected of having cancer, tumor marker levels being often elevated before appearance of any clinical evidence of cancer.

Many of the more clinically useful tumor markers identified over the last 10 years are mucins. A review of mucins as tumor markers has been edited by Feller W.F. et al, 1990, Immunology Series, 53, 631-672, and Bombardieri et al., p. 477-486, in: "Updating on tumor markers in tissues and in biological fluids. Basic aspects and clinical applications", Ballesta A.M. et al., 1993, published by Minerva Medica, Torino - Italy. Mucins are glycoproteins mainly characterised by a high molecular weight, generally above 500 kDa in non reducing and non denaturing conditions, a high carbohydrate content ranging from 50 to 80 % of the molecular weight, a characteristic carbohydrate and amino acid composition, the presence of O-glycans, a high density and a high viscosity. These glycoproteins do not precipitate in perchloric acid solution and generally bind to lectins having galactose, N-acetylgalactosamine and/or galactose-β-N-acetylgalactosamine specificities.

As regards lung cancer the following tumor markers are frequently used for surveillance, follow-up and monitoring of lung cancer: Neuron Specific Enolase (NSE), Carcinoembryonic Antigen (CEA) and Squamous Cell Carcinoma (SCC) . Among these markers NSE is very specific for SCLC, whereas the other two do not display a high sensitivity at an acceptable specificity level for NSCLC cancer types. These markers are often used in combination with other tumor markers, called proliferation markers, which show a high sensitivity but a low specificity for NSCLC cancer types. These proliferation markers are activated in many events involving cell proliferation and are not limited to cancer. Examples of such proliferation markers are cytokeratinic markers such as TPA (P. Oehr et al., p. 193-206 in "Serological Cancer Markers", Stewart Sell, 1992, The Humana Press, Totowa, NJ, USA), TPS (P. Oehr et al., same reference) and CYFRA 21. The latter marker, which is a soluble fragment of cytokeratin 19, shows in NSCLC cancer types, especially in squamous cells carcinoma, a better sensitivity than CEA and SCC, as reported by Hasholzner U. et al., p. 132-134 in: "Tumor associated Antigens, Oncogenes, Receptors, Cytokines in tumor Diagnosis and Therapy at the beginning of the Nineties. Cancer of the Breast. States and Trends in Diagnosis and Therapy", Klapdor R. et al., 1992, W. Zuckschwerdt Verlag, München - FRG.

For screening persons suspected of having lung cancer and monitoring lung cancer patients, it is at present necessary to determine the levels of multiple lung cancer markers such as those mentioned above, in order to have reliable data on the presence and the progression of lung cancer.

The problem to be solved by the present invention is to find a lung cancer marker which shows a high sensitivity for many types of lung cancer with an acceptable specificity level and replaces these multiple lung cancer markers for assessing the presence of lung cancer in persons suspected of having lung cancer and monitoring lung cancer patients.

This problem is solved by the invention as claimed in the appended set of claims.

The tumor marker of the invention gives better results than NSE or CYFRA 21 for monitoring patients having a NSCLC lung cancer of any type and for screening persons suspected of having lung cancer, the type of which is not known.

This tumor marker may be isolated from a lung tissue or a body fluid, such as blood or sputum, of a lung cancer patient or from a human lung tumor cell line. It may represent the complete lung cancer antigen which is present inside tumor cells, at the surface of these cells or is shed from these cells in a soluble form, or a sub-part thereof. Said sub-part may contain an epitope which is recognized by one of the monoclonal antibodies P14B4 and P6F7, but may also be derived from a different sub-part of the complete lung cancer antigen which does not contain an epitope recognized by one of these monoclonal antibodies. The complete soluble form of the lung cancer antigen will hereafter be referred to as "LCA".

LCA may conveniently be isolated and purified from body fluids, particularly sera, of lung cancer patients by a method comprising the steps of desalting, immunochromatography on a column comprising an antibody recognizing LCA, e.g. a column consisting of CNBr activated SEPHAROSE™ (Pharmacia, Uppsala, Sweden) coupled with such an antibody, and exclusion chromatography. An advantageous antibody for immunochromatography is a monoclonal antibody selected from the group consisting of monoclonal antibodies P14B4 and P6F7. Preferably the immunoaffinity step is performed in a buffer containing between 10 and 100 mM of magnesium ions and having a pH between 4.0 and 8.0, particularly between 4.5 and 6.5.

It was found that LCA is a glycoprotein recognized by monoclonal antibodies P14B4 and P6F7, which has the following properties:
- an apparent molecular weight between 630 and 690 kDa when determined by gel filtration in non denaturing and non reducing conditions,
- a molecular weight between 360 and 440 kDa when determined in denaturing and non-reducing conditions by SDS-PAGE,
- a molecular weight between 76 and 94 kDa when determined in presence of β-mercaptoethanol by SDS-PAGE,
- an isoelectric point between 9.5 and 9.7,
- no precipitation in a solution containing from 1 to 500 mM of perchloric acid,
- binding to the following lectins: concanavalin A (ConA), peanut lectin from Arachis hypogaea (PNA), Wheat Germ Agglutinin (WGA) and lectin from Sambucus nigra (SNA),
- inhibition of the binding to monoclonal antibody P14B4 in presence of PNA lectin,
- suppression of the immunoreactivity with monoclonal antibody P14B4 after treatment with pronase,
- reduction of the immunoreactivity with monoclonal antibody P14B4 after treatment with periodate, neuraminidase, O-glycanase, trypsin or chymotrypsin,
- no inhibition of the binding to monoclonal antibody P14B4 in presence of monoclonal antibodies B 72.3, NS 1116, M11 and OC 125, b-12 or B72.3, described by Gold D. V. et al., 1988, Tumor Biol., 9, 137-144, Koprowski H. et al., 1979, Somatic Cell. Genet., 5, 957-971, Bast R.C. et al., 1981, J.Clin. Invest., 68, 1331-37, Stähli C. et al.,1985, Experientia, 41, 1377-1381, and Johnson V.G. et al., 1986, Cancer Res., 46, 850-857, respectively, and
- the amino acid composition set out in Table 2 hereafter.

These properties indicate that LCA is a novel mucin.

The tumor marker of the invention may also be prepared from a human lung tumor cell line which constitutively expresses a lung cancer antigen recognized by a monoclonal antibody selected from the group consisting of monoclonal antibodies P14B4 and P6F7. Examples of such cell lines are Small Cell Lung Cancer Carcinoma cell line NCI-H69 (ATCC HTB-119) and Non Small Cell Lung Cancer Adenocarcinoma cell line CALU-3 (ATCC HTB-55 ). The tumor marker is then prepared by a method comprising the steps of
(a) cultivating a population of cells constitutively expressing the lung cancer antigen under conditions which permit the expression thereof,
(b) isolating the tumor marker from the culture and
(c) purifying the tumor marker isolated in step (b).

The lung cancer antigen may be expressed inside the cells, at the surface of the cells and/or may be shed from the cells into the culture medium. The step of isolating the tumor marker from the culture may include a lysis of the cells, e.g. by a sudden drop of the osmotic pressure, and/or the use of one or more proteases to release all or part of the lung cancer antigen from the surface of the cells.

Preferred lung cancer cell lines are those in which an appreciable quantity of the complete soluble form of the lung cancer antigen, LCA, is released into the culture medium. Examples of such cell lines are NCI-H69 and CALU-3 mentioned above. LCA may then conveniently be isolated and purified using the above described method comprising the steps of desalting, immunochromatography and exclusion chromatography.

The tumor marker of the invention may also be prepared in a transformed host using recombinant DNA technology, by a method comprising the steps of
(a) culturing a microorganism transformed with a DNA coding for said tumor marker under conditions favorable for expression,
(b) isolating the tumor marker from the culture and
(c) purifying the tumor marker isolated in step (b).

In this approach total RNA is extracted according to techniques well known in the art from a lung cancer cell line constitutively expressing the tumor marker, such cell lines NCI-H69 and CALU-3 mentioned above, and prepared by the guanidium / cesium method described by Glisin V. et al., 1974, Biochemistry, 13,2633-2637. Polyadenylated RNA strands are selected by chromatography on oligo(DT)-cellullose, as described by Aviv H. et al., 1972, Proc. Natl.Acad. Sci. USA, 69, 1408-1412. cDNA fragments are generated using techniques well known in the art, e.g. using the cDNA Synthesis System Plus (Amersham, Little Chalfont, UK) as prescribed by the manufacturer. A cDNA expression library is then constructed by inserting the cDNA fragments into the β-galactosidase structural gene lacZ of phage λgt11 (Amersham) and transforming E. coli cells, according to techniques well known in the art, described for example by J. Sambrook et al. in "Molecular cloning: a Laboratory Manual, 1989, Cold Spring Harbor Laboratory Press, NY, USA.

Purified LCA is deglycosylated by treatment for 3 hours at 25°C with trifluoromethane sulfonic acid and phenyl alanine, as described by Crepin M. et al., 1990, Biorheology, 27, 471-484. An polyclonal rabbit antiserum to deglycosyiated LCA is prepared according to techniques well known in the art. Briefly one New Zealand rabbit is injected with deglycosylated LCA, first in Freund's complete adjuvant, then, four weeks later, in Freund's incomplete adjuvant. The rabbit is bled one week later. The antiserum obtained is tested on immunoblots after SDS-polyacrylamide gel electropohoresis. Immunoglobulin fractions of this antiserum are prepared by ammonium sulfate precipitation and stored at - 20°C.

The cDNA library is immunologically screened by plating the cultures on nitro-cellulose membranes and probing with the polyclonal antibodies obtained above, as described by R.A. Young at al., 1983, 80, 1194-1198. Positive plaques are isolated, recloned and sequenced. A full length cDNA coding for the amino acid sequence of the tumor marker is thus isolated.

The full length cDNA is introduced into a expression vector and a host microorganism is transformed with the vector obtained.

The expression vector may be a phage, a viral vector or, advantageously, a plasmid. It is chosen according to the host microorganism.

The host microorganism may be any prokaryotic or eukaryotic cell capable of expressing and, advantageously, secreting into the culture medium, the peptidic part of the tumor marker. Preferably it should also be capable of glycosylating the tumor marker in a manner similar to that of native LCA.

Animal cell lines, such as for instance COS cell lines, are particularly interesting host microorgamsms. Suitable expression vectors include the pCMV4 and pCMV5 expression vectors described by Andersson S. et al., J. Biol. Chem., 264, 8222-8229, which contain the SV40 origin, the cytomegalovirus major immediate early gene promoter, and the human growth hormone fragment with transcription, termination and polyadenylation signals.

The tumor marker prepared from recombinant animal cell lines may conveniently be isolated and purified using the above described method comprising the steps of desalting, immunochromatography and exclusion chromatography.

The invention also relates to a monoclonal antibody recognizing a lung cancer antigen as defined above having an apparent molecular weight between 630 and 690 kDa when determined by gel filtration. This monoclonal antibody is useful notably in the diagnosis of lung cancer.

Such a monoclonal antibody may be obtained by performing the following operations:
- isolation of immunogen fractions containing this lung cancer antigen from tissues or sera of patients with different lung cancer histotypes,
- production of a large number of hybridoma cell lines producing monoclonal antibodies against these immunogen fractions, and
- screening of these cell lines based on selective reactivity with these immunogen fractions in relation to possibly interfering components of healthy donors sera and/or difference in immunoreactivity of undiluted sera between lung cancer patients and healthy donors.

The isolation of immunogen fractions containing this lung cancer antigen from tissues or sera of patients with different lung cancer histotypes, is performed by a method comprising a step of desalting, preferably by passing through a gel filtration material retaining small molecules such as, for instance, crosslinked dextran SEPHADEX™ G25 (Pharmacia, Upsala, Sweden) which retains proteins having a molecular weight under 5 kDa, and a step of exclusion chromatography on a column containing a gel filtration material, the exclusion characteristics of which are such that a protein having an apparent molecular weight between 630 and 690 kDa is allowed to go through the column without entering the gel particles, while most other proteins of smaller apparent molecular weight are retained. This gel filtration material is for instance SEPHACRYL™ S200 (Pharmacia) having a fractionation range of 5 to 200 kDa, or SUPEROSE™ (Pharmacia). It may be useful to add one or more steps for removing other known high molecular weight proteins, such as haptoglobins. Such steps are conveniently performed by immunochromatography, e.g. on a column comprising CNBr-activated SEPHAROSE™ (Pharmacia) coupled with a monoclonal antibody recognizing one of these high molecular weight proteins.

The production of hybridoma cell lines is carried out according to techniques well known in the art. Briefly mice are repeadly immunized with the immunogen fractions described above in an immunological adjuvant. Examples of suitable protocols are described by Chard. T., 1987, Laboratory Techniques in Biochemistry and Molecular Biology, vol. 6, Partz Elsevier, N.Y. USA. The mice are sacrificed and their spleens are fused with a myeloma cell line, as described by Köhler G. et al, 1975, Nature, 256; 495-497 and 1976, Eur. J. Immunol., 4, 511-519. Generated clones are grown in a culture medium suitable for growing hybridoma cells, as described in "Antibodies. A laboratory manual.", Harlocv et al., 1988, Cold Spring Harbor Laboratory, NY - USA.

The screening of the clones based on selective reactivity with the immunogen fractions in relation to possibly interfering components of healthy donor sera is advantageously performed by indirect ELISA.

In indirect ELISA the antigen, which is either a immunogen fraction isolated as described before, or a possibly interfering component of normal serum, is bound to the solid phase, e.g. adsorbed on a microtiter plate, and incubated with a labeled monoclonal antibody (produced by an hybridoma belonging to the population of clones to be screened), e.g. a horseradish peroxidase conjugated monoclonal antibody, non specific binding sites being blocked with a blocking solution, such as bovine serum albumin (BSA) in phosphate buffered saline, as described by Towbin et al, 1979, Proc. Natl. Acad. Sci. USA, 76, 4350-4354.The substrate is added and the color reaction develops. An alternative, which has the advantage of using only one labeled monoclonal antibody, is to use first a non labeled antibody and to make a second incubation with a labeled anti-immunoglobulin, e.g. a horseradish peroxidase-conjugated goat anti-mouse immunoglobulin, as described by Avrameas S. et al., 1978, Scand. J. Immunol., 8, Suppl. 7,7.

Clones selected in this way are those the monoclonal antibodies of which strongly react with the immunogen fraction and show no reactivity with other possibly interfering components of normal human serum.

The screening of the clones based on difference of immunoreactivity of undiluted sera between lung cancer patients and healthy donors advantageously comprises a dot immunobinding step and/or a Western blot step and a sandwich assay step.

In the dot immunobinding step serum samples from lung cancer patients and from healthy donors are adsorbed on a nitrocellulose membrane which, after washing with Tris-buffered saline (TBS), is incubated first for at least 10 minutes with a blocking solution, then overnight with hybridoma supernatant. After washing with TBS and saturation with the blocking solution for at least 10 minutes, another incubation is made with a secondary antibody labeled with a suitable enzyme. Coloured spots (dots) occur after addition of the appropriate substrate, the intensity of which is estimated semi-quantitatively with the naked eye or evaluated accurately by densimetry in a reflectance spectrophotometer. The secondary antibody is preferably an antibody conjugated with peroxidase, such as for instance a horseradish peroxidase-conjugated anti-mouse immunoglobulin, as described by Avrameas S. et al., 1978, Scand. J. Immunol., 8, Suppl. 7, 7. The reaction is then developed using a solution containing hydrogen peroxide, as described by Hawes R. et al., 1982, Anal. Biochem., 227, 142-147.

MAbs selected in this way are those which give a significantly higher signal for lung cancer patients sera than for healthy donors sera.

In the Western Blot step proteins are separated according to their molecular weight by SDS-PAGE electrophoresis, as described by Laemmli U.K., 1970, Nature, 227, 680-685, and transferred to a nitrocellulose filter according to the method of Towbin et al., 1979, 76, 4350-4354. The developing procedure used, is that described above for dot immunobinding.

MAbs selected in this way are those which give a significantly higher signal for lung cancer patients sera than for healthy donors sera, this signal corresponding to a high molecular weight protein.

In double determinant ELISA monoclonal antibodies are first bound to the solid phase, e.g. adsorbed on a microtiter plate, and then incubated with the antigen. After washing, a second incubation is made with a labeled monoclonal antibody, e.g. a peroxidase conjugated monoclonal antibody.

In the sandwich assay step selected MAbs are evaluated in different couples in a preliminary clinical study using a sandwich immunoassay on microtiter plates to measure the circulating antigen levels in sera from a group of healthy blood donors and a group of lung cancer patients. Monoclonal antibodies which allow a good discrimination between these two groups are thus selected.

Examples of advantageous monoclonal antibodies which were obtained using the above described methodology of screening are monoclonal antibodies P14B4 and P6F7.

The invention also relates to a method for diagnosing lung cancer which comprises quantitating the above defined tumor marker in a sample derived from a body fluid or a lung tissue of an individual suspected of having lung cancer. Due to the relatively low concentrations of this tumor marker in body fluids and lung tissues, this method must show a high sensitivity.

This method is preferably an immunological method, i.e. an immunoassay, comprising the step of exposing a sample derived from a body fluid or a lung tissue of an individual suspected of having lung cancer to an antibody recognizing the above defined tumor marker. This antibody is either a polyclonal antibody which may be raised against the purified tumor marker according to techniques well known in the art (cf "Antibodies. A Laboratory Manual", Harlow et al., 1988, Cold Spring Harbor Laboratory, NY- USA) or a monoclonal antibody which may be raised against the purified tumor marker or an immunogen preparation containing the tumor marker and selected for its specificity and/or high affinity for the tumor marker according to conventional techniques such as those mentioned hereinbefore. Particularly interesting such monoclonal antibodies are those described above.

As will be recognized by those in the art, numerous types of immunoassays are available for use in the present invention. For instance homogeneous and heterogeneous assays, direct and indirect binding assays, competitive assays, sandwich assays and more generally all assays which correspond to the first four groups of immunoassays sorted by Gosling J.P., 1990, Clin. Chem., 36, 8, 1408-1427. These immunoassays are well known in the art and described in numerous publications, e. g. "Antibodies. A Laboratory Manual", Harlow et al., 1988, Cold Spring Harbor Laboratory, NY- USA.

The antibody recognizing the tumor marker, the tumor marker or another component of the test kit may carry a label depending upon their application. A "label" means here a molecule which provides, directly or indirectly, a detectable signal. Various labels may be employed, such as radionucleotides (e.g. ¹²⁵I, ¹³¹I, technetium, indium, ³H and ¹⁴C), fluorescers, chemiluminescers, enzymes (e. g. peroxidase, alcaline phosphatase, β-D-galactosidase, glucose oxidase, glutamate decarboxylase and β-amylase), enzyme substrates, cofactors and inhibitors, particles (e.g. colloidal gold particles), combinations of ligands and receptors (e.g. streptavidin and biotin) and the like. Enzymatic labels are advantageous because they allow a high sensitivity, comparable to that of radioactive labels, do not require particular safety precautions and can be used in commercially available automated systems, such as for instance COBAS® Core Roche immuchemistry analyser described by Henne V. and Maurer A., 1993, The International Journal of Biological Markers, 8, 2, 133-137. An enzymatic label which is often used is horseradish peroxidase.

Particularly interesting immunoassays are sandwich assays and competitive inhibition immunoassays which use at least one monoclonal antibody as defined above.

The sandwich assay may be a homosandwich assay, a heterosandwich assay or a lectin-immunometric assay.

In a homosandwich assay the same monoclonal antibody is used as a solid phase capture reagent (catcher) and as the labeled tracer reagent (tracer) to form the homosandwich with the tumor marker. In order to allow the formation of the trimeric homocomplex the tumor marker must contain at least two identical antigenic groups which are stereochemically sufficiently apart to favour the immunobinding reaction. This condition is satisfied for a large number of monoclonal antibodies raised against the tumor marker, which like other mucins, generally has a repetitive structure, hence repeated epitopes.

In a heterosandwich assay the catcher and the tracer are two different antibodies, one of which is a monoclonal antibody as defined before and the other is either another such monoclonal antibody recognizing a different epitope of the tumor marker or a polyclonal antibody. An advantage of this format compared to the homosandwich assay is that it has a better specificity and avoids the potential self-quenching and stereochemical inhibition of homosandwich assay which may, in certain cases, result in underestimation of the concentration of the tumor marker.

A lectin-immunometric assay is a variant of the above described heterosandwich assay in which one of the component of the ternary sandwich complex is a carbohydrate binding lectin. This lectin may be chosen among the lectins which bind to the tumor marker, and preferably among those which have a strong affinity for the tumor marker. For instance when the tumor marker is LCA, a preferred lectin is concanavalin A which is advantageously used as a catcher in a two-step assay, monoclonal antibody P14B4 conjugated to horse radish peroxidase being the tracer.

The sandwich assay may include one or two steps.

In a one-step sandwich assay the sample or standard is incubated in a buffer with the solid phase (e.g. a bead) to which the antibody or lectin is bound (the catcher) and with the labeled antibody or lectin (the tracer). The catcher and the tracer bind to the tumor marker, a "sandwich" being formed between the catcher, the tumor marker and the tracer. Unbound sample and tracer are washed off. The signal detected is proportional to the amount of bound tracer which corresponds to the amount of tumor marker present in the sample.

In a two-step sandwich assay the sample or standard is first incubated in a buffer with the catcher. The catcher binds with the tumor marker. The unbound sample is washed off. A second incubation is then carried out in a buffer with the tracer. The latter binds to the tumor marker, a "sandwich" being formed between the catcher, the tumor marker and the tracer. The signal detected is proportional to the amount of bound tracer which corresponds to the amount of tumor marker present in the sample.

Sandwich assays require the drawing of standard curves where the tumor marker concentration is plotted against the detected signal. Such standard curves remain remarkably stable when using horseradish peroxidase as an enzymatic label and an automated immunoanalyzer, such as COBAS® Core Roche immunochemistry analyzer (Cf Henne V. and Maurer A., above reference).

The competitive inhibition immunoassay involves primarily two components, namely the tumor marker and a monoclonal antibody as defined above, wherein either one of these is labeled and the other component is immobilized on solid phase for ease of separation of the reactants prior to signal measurement. The sample containing the analyte inhibits the antigen-antibody formation to a degree that is proportional to the concentration of the analyte.

If the monoclonal antibody is immobilized on solid phase, the test procedure may be as follows. The sample or standard is incubated in a buffer with the solid phase coated with the antibody and the labeled tumor marker for a sufficient time to reach equilibrium. The labeled tumor marker competes with the non labeled tumor marker of the sample or standard for binding to the solid phase antibody. Material which is not bound to the solid phase is washed off. The amount of bound labeled tumor marker is determined by measuring the signal before and alter the incubation and washing off. The amount of labeled tumor marker which has been displaced during incubation is proportional to the concentration of the tumor marker in the sample.

If the tumor marker is immobilized on solid phase, the test procedure may be as follows. The sample or standard is incubated in a buffer with the solid phase coated with the tumor marker and the labeled antibody for a sufficient time to reach equilibrium. The tumor marker on the solid phase competes with the tumor marker in the sample or standard for binding to the labeled antibody. Material which is not bound to the solid phase is washed off. The amount of bound labeled antibody is determined by measuring the signal before and alter the incubation and washing off. The amount of labeled antibody which has been displaced during incubation is proportional to the concentration of the tumor marker in the sample.

The above described procedures allow the determination of the amount of tumor marker in a sample without prior drawing of a calibration curve. They require however relatively long incubation times in order to reach equilibrium and these may be unpractical, particularly in hospitals where a large number of assays must be conducted using automated immunoanalyzers.

A convenient alternative is to draw, with the help of standards containing known amounts of the tumor marker, a standard curve plotting the detected signal alter a given incubation time as a function of the tumor marker concentration. The tumor marker concentration in unknown samples can then be determined by measuring the signal, even without equilibrium at the end of the incubation time.

All sandwich assays and competitive inhibition assays described above involve the step of incubating a monoclonal antibody as described above and the tumor marker. It is advantageous to perform this step in a buffer containing between 10 and 100 mM of magnesium ions and having a pH between 4.0 and 8.0, preferably between 4.5 and 6.5.

A preferred heterosandwich two-step immunoassay will now be described in greater details. This assay is designed to be run on COBAS® Core Roche immunochemistry analyzer described by Henne V. and Maurer A., 1993, The International Journal of Biological Markers, 8, 2, 133-137.

A first incubation of 30 minutes at 37 °C under permanent shaking is performed with a 5 µl sample, a polystyrene bead coated with monoclonal antibody P14B4 b as described by R.H. Burdon et al. in "Laboratory Techniques in Biochemistry and Molecular Biology", P. Tijssen, 15, 297-328, Publisher Elsevier,New York-USA, and 300 µl of a buffer solution, hereafter referred to as "test buffer", containing 25 mM magnesium chloride buffer, 20 mM sodium acetate, pH 5.0, 1.3 mg/l bromophenol blue and O.1 % Kathon (an antimicrobial reagent available from Rohm et Haas, Via Vittor Pisani, Milano, Italy). Unbound components of the test buffer and the sample are washed off with 3-5 ml of distilled water.

250 µl of a buffer solution, hereafter referred to as "conjugate buffer", containing monoclonal antibody P6F7 conjugated to horseradish peroxidase, 20 mM MES (2-morpholino-ethylsulphonic acid) pH 6.0, and 50 µg/mg of non specific mouse monoclonal antibody are added to the washed solid phase and a second incubation is performed with permanent shaking for 30 minutes at 37 °C. Unbound components of the conjugate buffer are washed off with 3-5 ml of distilled water. 250 µl of substrate 3,3',5,5'-tetramethyl benzidine (TMB) are added and the development reaction proceeds for another 15 minutes at 37 °C, with permanent shaking.

Specifically bound conjugate is then quantified by measuring the absorption at 650 nm. The tumor marker concentration can be calculated from a stored or a newly determined calibration curve.

The above described immunoassay can also be run manually by using a 50 µl sample (5 µl Sample 10-fold diluted) and 250 µl of test buffer instead of the above specified amounts in the first incubation and subsequently proceeding as described above, but in stopping the development reaction by addition of 1ml of 1N sulfuric acid and measuring the absorption at 450 nm.

The invention also relates to reagents and kits for performing the above described immunoassays.

These assays generally involve a step using a reagent containing the above defined monoclonal antibody and a buffer and/or an inert carrier.

The buffer must allow preservation of the monoclonal antibody over a long period and enhance the immunobinding reaction between the monoclonal antibody and the tumor marker. An example of an interesting such buffer is a buffer which contains between 10 and 100 mM of magnesium ions and has a pH between 4.0 and 8.0, preferably between 4.5 and 6.5.

The inert carrier is made of a material which does not react with any substance used in the immunoassay but due to its affinity for the monoclonal antibody immobilizes the latter and thus facilitates separation of the immunocomplex prior to signal measurement. Such materials are known in the art and have been described in numerous publications, e. g. "Antibodies. A Laboratory Manual", Harlow et al., 1988, Cold Spring Harbor Laboratory, NY- USA. Examples of convenient inert carriers are glass, polyacrylamide, polystyrene or latex beads.

Test kits for performing these assays usually comprise the above defined reagent, a label and instructions for use.

The label may be linked to an antibody recognizing the tumor marker, to tumor marker present in the kit or to another component of the kit. It allows quantification of the amount of tumor marker recognized by the monoclonal antibody of the above described reagent.

A particularly interesting kit is one in which the reagent contains monoclonal antibody P14B4 and the label is linked to monoclonal antibody P6F7.

The invention also concerns an imaging agent for lung cancer, i.e. an agent for visualizing in vivo lung cancer cells, said imaging agent comprising a monoclonal antibody as defined above linked to a detectable label such as a radionuclide, e.g. ¹²⁵1, ¹³¹I, technetium or indium. Such an imaging agent would be useful for detecting lung tumors in situ by a method including the steps of identifying person suspected of having a tumor, introducing the imaging agent into the lung tissues and detecting (e.g., by radioimaging, using scintigraphy) the presence of the detectable label bound to a lung tissue, a high level of such label bound to a given site being indicative of a tumor at that site. Using such an imaging method permits a non-invasive determination of the presence, location or absence of LCA expressing tumor in a person, which would be particularly useful for monitoring the condition of a patient being treated for a tumor known to express LCA.

In another aspect the invention relates to an immunotoxin comprising a monoclonal antibody as defined above conjugated to a toxin molecule. Such conjugation may be accomplished by known chemical methodology, or, if the toxin is a protein, by means of genetically engineering a hybrid DNA molecule encoding both the toxin and LCA-binding portion of the antibody as a single polypeptide: expression of this recombinant DNA molecule would result in an immunotoxin in which the antibody portion is linked to the toxin portion by a peptide bond. Examples of naturally-occurring proteinaceous toxins that could be incorporated into the immunotoxin of the invention include diphtheria toxin, Pseudomonas exotoxin A, ricin and other plant toxins such as abrin, modeccin, volkensin, and viscumin, cholera toxin (produced by Vibrio cholerae bacteria), Shiga toxin (produced by various strains of Shigella bacteria), the so-called "Shiga-like" toxins (produced by E. coli and other enteric bacteria), Salmonella heat-labile enterotoxin and E. coli heat-labile enterotoxin. Non-proteinaceous toxins include known cytotoxic anticancer agents such as doxorubicin, as well as α-emitting radionuclides such as astatine and β-emitting nuclides auch as yttrium. This immunotoxin is useful for targeting and killing tumor cells which express LCA on their surfaces.

The invention also concerns a vaccine for immunizing a human against lung tumors which express the above defined tumor marker, which vaccine includes this tumor marker , in a pharmaceutically acceptable carrier. This vaccine preferably includes an adjuvant such as Freund's adjuvant to enhance the recipient's immune response to the vaccine. Formulation of this vaccine may be done by the person skilled in the art according to conventional techniques well known in the art.

The invention will be further illustrated by the following examples. The following description will be better understood by referring to Figures 1 to 4.

Figure 1 represents the LCA levels determined by measurement of optical density at 450 nm using a heterosandwich two-step immunoassay with monoclonal antibody P14B4 as a catcher and monoclonal antibody P6F7 conjugated to horseradish peroxidase as a tracer, for serum samples of a group of 65 healthy donors and a group of 95 lung cancer patients with different types of lung cancer.

Figure 2 represents a micrograph of human small lung cancer adenocarcinoma cell line NCI-H69 analyzed by immunocytofluorescence using monoclonal antibody P14B4 (magnification 250X).

Figures 3 and 4 represent micrographs of lung adenocarcinoma cells (magnification 500X) and normal alveolar epithelium cells (magnification 300X), respectively, analyzed by immunohistochemistry using monoclonal antibody P14B4.

### Example 1: Isolation of immunogen fractions and production of hybridoma cell lines producing monoclonal antibodies against these immunogen fractions.

### 1) Isolation of immunogen fractions

The immunogen was obtained from biopsies of cancer patients having bronchio-alveolar carcinomas. The tissue was first frozen with liquid nitrogen, then homogenized and suspended in a buffer containing PMSF (phenyl-methyl-sulfonyl-fluoride) as a protease inhibitor. The suspension was stored for 2 hours at 4°C and then desalted by passing through a SEPHADEX™ G25 column (Pharmacia, Uppsala, Sweden). The desalted fraction was fractionated by exclusion chromatography on a SEPHACRYL™ S 200 column (Pharmacia) having a fractionation range of 5 000 - 200 000 Da. The quantity of proteins in the fractions was measured based on the optical density at 280 nm. The void volume fractions contained about 6-7% of the total proteins applied to the SEPHACRYL™ S 200 column. The unbound fraction was recovered and used for the immunization of mice as described below.
In another experiment the immunogen was obtained from a pool of sera of patients with different lung cancer histotypes. A high molecular weight fraction thereof was prepared using the same steps of desalting and exclusion chromatography as described above. The void volume fractions were injected into an immunochromatography column consisting of CNBr-activated SEPHAROSE™ CL-4B (Pharmacia) coupled with antihaptoglobin polyclonal antibodies. This immunochromatography step was repeated. The immunogen fraction stripped of haptoglobin was recovered and also used for the immunization of mice as described below.

### 2) Production of hybridomas

Female Balb/c mice of three weeks old were immunized with each of the above two immunogen fractions derived from tissues or sera. Donor mice were primed intraperitoneally with 100 µg of antigen emulsified in Freund's complete adjuvant. After 14 days the mice received an additional 100 µg dose of antigen in incomplete adjuvant. This procedure was repeated more than two times. Two days alter the last injection, performed by injecting (intraperitoneally) 100 µg of antigen in Freund's incomplete adjuvant and (intravenously) 100 µg of antigen diluted in PBS, the mice were sacrified and their spleen cells were fused with PAI-0 myeloma cells (Research Disclosure, 1982, 21713), as described by Köhler G. et al, 1975, Nature, 256, 495-497 and, 1976, Eur. J. Immunol., 4, 511-519.
The generated clones were grown in RPMI 1640 medium (Gibco, P.O. Box 35, Paisley, Scotland, UK) containing 20% fetal calf serum and sodium pyruvate, L-glutamine, penicillin/streptomycin and hypoxantine-aminopterin-thymidine at a final concentration of 1% (hereatter referred to as HAT medium). Hybridomas were subcloned by dilution. Monoclonal antibodies (MAbs) isotype was determined using the mouse monoclonal antibody isotyping kit (Amersham- Little Chalfont, UK - ref. RPN 29), and MAbs purification was performed from hybridomas supernatants by G-protein SEPHAROSE™ Column.
405 and 315 clones were thus obtained, for immunization with the immunogen fraction derived from tissues and the immunogen fraction derived from sera, respectively. The population of those 720 clones was used in subsequent screenings.

### Example 2: Screening of these hybridoma cell lines in order to select particularly interesting monoclonal antibodies for an immunoassay for detecting and quantifying in a body fluid a soluble lung cancer antigen contained in these immunogen fractions.

### 1) First screening by ELISA based on selective immunoreactivity with the immunogen in relation to other possibly interfering components of healthy donors sera.

Indirect ELISA was performed by coating the crude antigen (5 µg/ml of either the isolated immunogen or a possibly interfering component of a normal serum) on a 96 well polyvinyl chloride microtiter plate at 4°C overnight. Non specific binding sites were blocked by 10% fetal calf serum (FCS) in phosphate buffer saline (PBS) (blocking solution) for 30 minutes at 37°C, and then the wells were incubated for 1 h at 37°C with the MAbs (produced by the 720 clones to be screened).
After washing with PBS, the plates were incubated with horseradish peroxidase-conjugate goat anti-mouse immunoglobulin (Amersham - ref. NA 9310) linked in blocking solution (1/1000) for 1 h at room temperature. 3,3',5,5'- tetramethyl benzidine (TMB) was used as substrate. The reaction was stopped by the addition of 1 N sulfuric acid (stopping solution), and the optical density was read at 450 nm in a microtiter plate reader.

### Results

720 clones were screened in the ELISA assay. The strategy adopted was based on a double simultaneous first screening: the selected clones were positive versus their related immunogen fraction and negative versus other possibly interfering components of the normal sera. In this way 114 clones were chosen because of the strong reactivity of their MAbs towards the immunogen fraction; none of these MAbs showed reactivity towards other possibly interfering components of normal human serum, such as haptoglobins or other known circulating glycoproteins.

### 2) Second screening by dot immunobinding assay (dot blot), based on difference of immunoreactivity of undiluted sera between lung cancer patients and healthy donors

Serum (undiluted) or antigen (24 µg/ml of one of the immunogen fractions described above used as positive control) samples were adsorbed as a spot of 1 µl volume on a nitrocellulose filter. After washing with 50 mM Tris-buffered saline (TBS) pH 7.4, 200 mM NaCl , an incubation was performed for 15 minutes with a blocking solution (10% FCS in TBS).
The filter was incubated with hybridoma supernatant overnight. After washing with TBS and saturation with the blocking solution for 15 min, a second incubation was made a peroxidase labeled goat anti-mouse Ig (Amersham - Little Chalfont, UK- ref. NA 9310) diluted 1/500 in a blocking solution. The reaction was developed by adding a 4-chloro-1-napthol (0.3% w/w in methanol) in TBS containing from 0.01% to 30% of hydrogen peroxide, as described by Hawes R. et al, 1982, Anal. Biochem., 227, 142-147.
A panel of 26 MAbs showing a higher signal for lung cancer sera than for healthy patients, was thus selected.

### 3) Third screening by Western Blot, based on differences in immunoreactivity of sera between lung cancer patients and healthy donors.

Serum proteins separated by SDS-PAGE gel electrophoresis as described by Laemmli U.K, 1970, Nature, 227, 680-685, were transferred to a nitrocellulose filter according to the method of Towbin et al., 1979, Proc. Natl. Acad. Sci. USA, 76, 4350-4354. The developing procedure used, is that described above for dot blot analysis.
Among the 26 MAbs analyzed by Western Blot, seven showed a significant higher signal for lung cancer patients sera than for healthy donors sera, this signal corresponding to a high molecular weight protein (about 400 kDa).

### 4) Fourth screening by a sandwich assay using different couples of antibodies:

### Double determinant ELISA

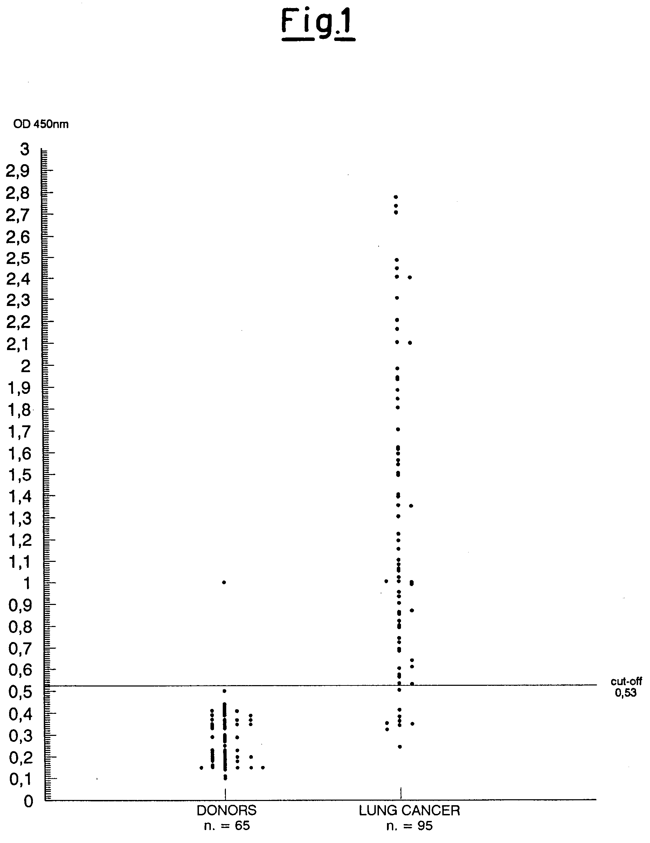

Monoclonal antibodies (2.5 µg/ml) in 20 mM sodium acetate buffer (pH = 5.5), were absorbed to a 96 well microtiter plate for 3 h at room temperature. The wells were blocked with 5% bovine serum albumine (BSA) in phosphate buffered saline (PBS) for 3 h at 37°C and then incubated with the antigen diluted in PBS for 1 h at 37°C. After washing, the wells were incubated with horseradish peroxidase-conjugated-MAbs for 1 h at room temperature, washed and the reaction was developed with TMB, blocked with 1N sulfuric acid and monitored by measuring the absorbance at 450 nm.
On the basis of different patterns of immunoreactivity of these 7 selected MAbs it was possible to develop a preliminary sandwich immunoassay on microtiter plates. MAbs were evaluated in different couples to measure the circulating antigen levels in sera from 65 healthy blood donors, 44 patients with inflammatory diseases and 95 patients with lung cancer at different stages. A significant normal/cancer discrimination was observed in those tests which included the following monoclonal antibodies: P14B4 (IgG1) and P6F7 (IgG1) which corn from hybridomas obtained by immunization with the immunogen fraction derived from tissues and N6E10 (IgG1) which comes from a hybridoma obtained by immunization with the immunogen fraction derived from sera. The hybridomas producing MAbs P14B4 and P6F7 were deposited in accordance with the Budapest Treaty on May 4, 1994 at the DSM under the numbers ACC2170 and ACC2171, respectively.
The evaluation of the best combination of the selected MAbs to determine the circulating lung cancer antigen (LCA) was performed in an automated random access heterosandwich two-step immunoassay, which is a modification of the MCA-EIA Roche format described by Müller-Brand J. et al., 1990, p 157-160 in: "Recent Results in Tumor Diagnosis and Therapy", Klapdor R., W. Zuckschwerdt Verlag, München. In this test one of the MAb is used as a catcher, the other as a tracer.
The highest performances were obtained using P14B4 as a catcher and P6F7 conjugated to horse radish peroxidase (P6F7-HRP) as a tracer. The immunoassay used is close to the immunoassay described in Example 6 hereafter. Figure 1 shows the LCA levels determined by measurement of the optical density values at 450 nm for serum samples of a group of 65 healthy donors and a group of 95 lung cancer patients having different types of lung cancer. As shown on this figure this test allows to clearly discriminate between the two groups with a cut-off value of 0.53 (represented by a horizontal straight line) corresponding to a specificity level of 98%.

### Example 3: Visualization of LCA by indirect immunocytofluorescence of human cell lines. Immunohistochemical localization of LCA in normal and cancer cells.

### 1) Human cell lines cultures

The human lung carcinoma cell lines CALU-3 (NSCLC adenocarcinoma), and NCI-H69 (SCLC adenocarcinoma) were obtained from the American Type Culture Collection under references ATCC HTB-119 and HTB-55, respectively. Cell cultures were maintained in RPMI 1640 medium (Gibco, P.O. box 35, Paisley, Scotland) supplemented with 10% of heat-inactivated fetal calf serum and, at final concentrations of 1% (w/w), sodium pyruvate, L-glutamine and penicillin-streptomycin.

### 2) Analysis by indirect immunocytofluorescence of human cell lines

The human continuous cell lines were grown in the culture medium previously described and placed into chamber slides (Lab-tek Inc., Naperville Illinois, USA). The slides were fixed in acetone for 10 minutes rinsed in PBS, then incubated with a blocking solution (20% FCS in PBS) for 30 minutes at 37°C. The incubation with MAb P14B4 (50 µg/ml) in the blocking solution was performed at room temperature for 30 minutes.
After washing at 4°C, the slides were incubated for 1 h at 4°C with fluorescein isothiocyanate (FITC) -labeled goat anti-mouse immunoglobulin (Boehringer Mannheim - ref. 8214462) diluted 1/100 in the blocking solution. The slides were extensively washed in PBS, after that a drop of 10% glycerol in PBS was applied to glass slides, then after adding a coverslip viewed using a Leitz Diaplan fluorescence microscope.

### 3) Analysis by immunohistochemistry of human tissues

4-6 µm thick sections of cancerous tissue and adjacent normal tissue obtained from NSCLC adenocarcinoma patients, which had been fixed in 10 % buffered formalin and paraffin embedded, were submitted to immunoperoxidase staining using the Dako LSAB™ kit Peroxidase Universal K679, available from Dako, Carpinteria, Ca - USA. This kit uses the avidin biotin peroxidase staining technique described in "Handbook Immunochemical Staining Methods" Naish S.J., 1989, published by Dako, Carpinteria, Ca, USA. All samples were treated with 0.05% (w/v) trypsin for 9 minutes at 37°C. For specific reaction with MAb P14B4 (primary antibody) the undiluted supernatant was used. The sections were counterstained with Mayer's hematoxyline (Sigma, Saint Louis, MO, USA- ref. MHS-32) and AEC (3-amino-9-ethylcarbazole) as chromogen was employed.

### 4) Results

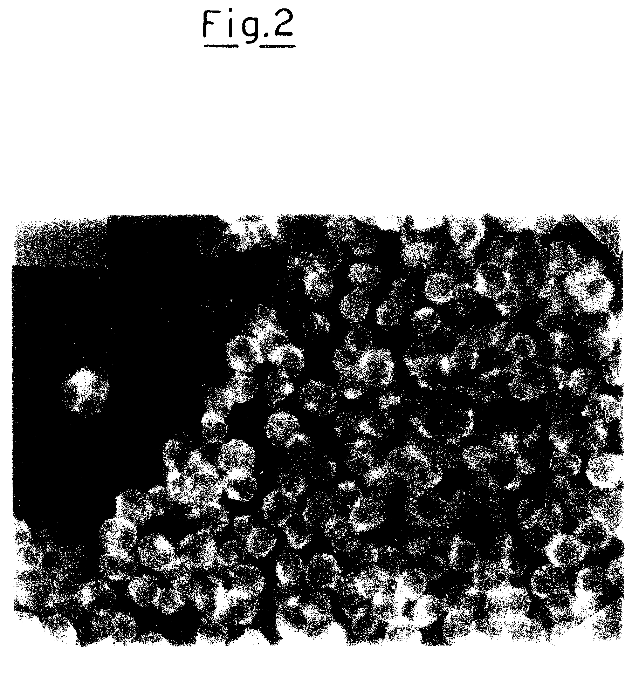

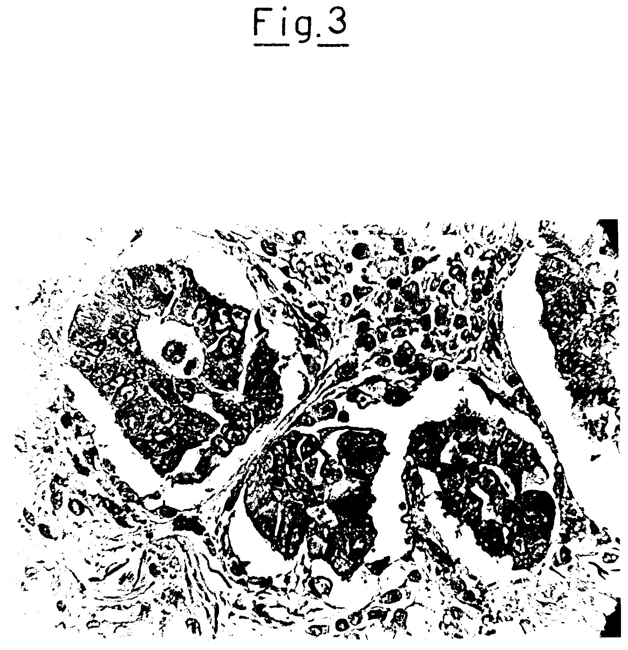

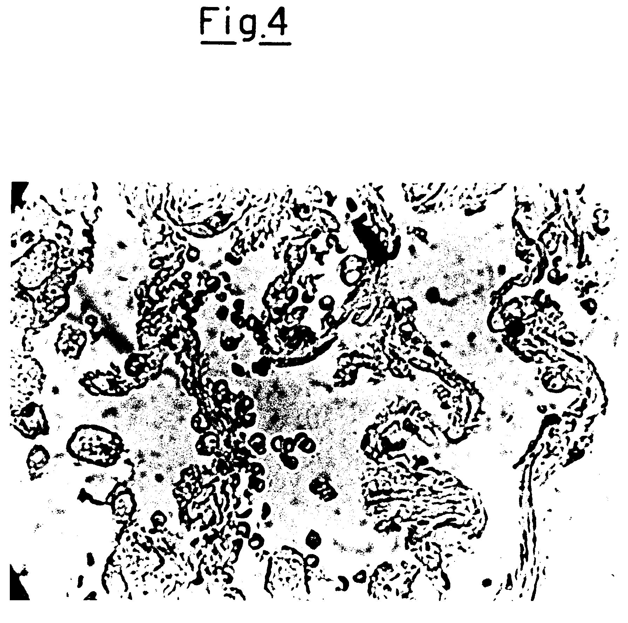

The above studies by means of immunocytofluorescence and immunohistochemistry techniques showed that, for these cell lines and tumorous tissue, the lung cancer antigen is mainly localized on the cell membranes and in the cytoplasm.
The immunocytofluorescence staining showed a significant positive reaction in the cells of small cells adenocarcinoma cell line NCI-H69 (Fig. 2) and squamous carcinoma cell line CALU-3.
The immunoperoxidase staining of the paraffin embedded tissues revealed an intense cytoplasmatic staining in well-differentiated lung adenocarcinoms cells (Fig. 3); on the other hand no positive reaction was observed in normal alveolar epithelium cells (Fig. 4).
The visualization of the lung cancer antigen in human cells and tissues using the monoclonal antibody of the invention allows to differentiate between cancer cells and normal cells.

### Example 4: Isolation and purification of LCA from sera of lung cancer patients.

Sera of lung cancer patients were desalted by gel filtration on SEPHADEX G-25 (Pharmacia). The desalted material was loaded on a MAb P14B4 immunoaffinity column prepared by coupling 8 mg of MAb / gr of CNBr-activated SEPHAROSE™ CL-4B (Pharmacia). The antigen was eluted with a 0.1M glycine/HCl pH 2.5 buffer containing 0.05 M magnesium chloride. The eluted fractions were collected, immediately neutralized with 1M Tris-HCl, pH 9.0 and concentrated in a stirred ultrafiltration cell (e.g. an Amicon with a YM 10 membrane, available from Amicon, Beverly, Ma, USA). Then, the antigen was applied to a gel filtration chromatography HPLC column WS804F (Millipore Corporation,Waters Chromatography Division, Milford, Ma, USA) having a fractionation range between 500 Da and 600 kDa, which had been first equilibrated with a 0.05 M sodium phosphate buffer, pH 7.0, containing 0,15 M NaCl. Elution was performed at room temperature. LCA contained in the void volume fraction was used for the biochemical characterization set out in example 5.
At each step in the above protocol the LCA content was measured with an ELISA assay using MAbs P14B4 and P6F7-HRP; the purity of the LCA was evaluated by SDS-PAGE both in presence of and without β-mercaptoethanol, followed by Coomassie staining and Western Blot analysis.
In another experiment LCA was isolated and purified using the same steps as described above but with an immunoaffinity column comprising MAb N6E10 instead of MAb P14B4. Similar results were obtained as regards the purity of LCA.

### Example 5: Biochemical characterization of LCA

Biochemical characterisation of LCA was carried out on purified LCA obtained as described in Example 4.

### 1) Analysis by gel filtration and SDS-PAGE / Western Blot

The apparent molecular weight of LCA was determined using gel filtration HPLC column 4000 SW (available from TosoHaas, Mahwah, NJ, USA) in a 50 mM sodium phosphate buffer, pH 7.0, containing 50mM sodium chloride. This apparent molecular weight thus measured was between 630 and 690 kDa.
LCA was first subjected to polyacrylamide gel electrophoresis in presence of SDS (denaturing conditions), as described by Laemmli U.K. et al., 1970, Nature, 227, 680-685, with and without β-mercaptoethanol (an agent capable of reducing disulphide bridges) at a final concentration of 5.5% (v/v), then to a Western Blot, as described by Towbin H. et al., 1979, Proc. Acad. Sci. USA, 76, 4350-4354.
In non reducing conditions two bands were observed corresponding to a molecular weight of 400 ± 40 kDa, whereas, in presence of β-mercaptoethanol, only one band could be seen corresponding to a molecular weight of 85 ± 9 kDa. No contaminating protein was detectable.

### 2) One-dimensional isoelectric focusing.

One-dimensional isoelectric focusing was performed as described by Torgny Laas, 1989, p. 376-401 in "Protein purification. Principles, High Resolution Methods and Applications", Jan-Christer Janson et al., VCH Publishers, N.Y., USA.
The gel showed only one band corresponding to an isoelectric point of 9.6 ± 0.1.

### 3) Determination of the amino acid composition

The amino acid composition of purified LCA antigen was determined according to a modification of the method described by Spackman D.H. et al, 1958, Analyt. Chem., 30, 1190.
Briefly the purified antigen sample was divided into 3 vials and subjected to hydrolysis under the following conditions:
(a) incubation for 24 h at 110 °C after addition of 1ml 6N HCl
(b) incubation for 72 h at 110 °C alter addition of 1 ml 6N HCl
(c) incubation for 24 h at 110 °C after oxidation with performic acid and subsequent addition of 1ml 6N HCl
The amino-acid composition was determined with a Liquimat III amino-acid analyzer (available from Kontron AG., Zürich, Switzerland).
The amino-acid composition is set out in Table 2 hereafter.
This table takes into account a correction of the losses during hydrolysis.Due to overlapping with an impurity the amount of cysteine could not be determined.

### 4) Perchloric acid precipitation

This treatment of the antigen was performed at different concentrations of perchloric acid (1-500 mM) at 4°C for 30 min, as described by Sheer G.D.et al, 1988, Cancer Res., 48, 6811-6818. Evaluation of each sample, pellet and supernatant obtained after centrifugation at 1400 r.p.m. for 10 minutes and dialysis against PBS, was made in ELISA and dot blot assays using MAbs P14B4 and P6F7-HRP.
The purified antigen seemed to behave as a mucin-like glycoprotein since, in a perchloric acid precipitation treatment, no precipitation occurred at any perchloric acid concentration between 1 and 500 mM. Mucin-like Carcinoma associated Antigen (MCA), described by Stähli C. et al., 1985, Experientia, 41, 1377-81, used as positive control, showed the same behaviour.

### 5) Lectin binding assay

Reaction with lectins was made in incubating overnight at 4°C, as described by Craig G. et al, 1990, Methods in Enzymology, 182, 529., purified LCA bound to nitrocellulose and the following biotin labeled lectins (available from Boehringer-Mannheim, Mannheim, FRG): lectin from Aleuria aurantia (AAA), concanavalin A (ConA), peanut lectin from Arachis hypogaea ( PNA), soybean agglutinin (SBA), wheat germ agglutinin (WGA), lectin from Sambucus nigra (SNA), lectin from Maackia amurensis (MAA), at the concentration of 25 µg/ml in a 0.05% Triton X100 buffer. The binding of lectins to the antigen was detected using steptravidin - peroxidase (Sigma, Saint Louis, MO, USA) at 1/500 dilution.
Purified LCA was found to be able to bind with the following lectins: concanavalin A (ConA), peanut lectin from Arachis hypogaea (PNA), Wheat Germ Agglutinin (WGA) and lectin from Sambucus nigra (SNA). Based on this finding it seems likely that LCA contains galactose, N-acetylgalactosamine, alpha-mannose, alpha-glucose and sialic acid residues.

### Example 6: Biochemical characterisation of the epitope recognised by monoclonal antibody P14B4.

The characterization of the epitope recognized by MAb P14B4 was performed by submitting purified antigen LCA obtained as described in Example 4, to various treatments with the following agents capable of modifying carbohydrate or protein structure: sodium periodate, neuraminidase, N-and O-glycanase and proteases, and then determining the immunoreactivity of the thus treated LCA by Western Blot and/or an ELISA assay using MAb P14B4 on the solid phase and P6F7-HRP as conjugate antibody. In the Western Blot technique proteins are separated by SDS-PAGE gel electrophoresis as described by Laemmli U.K, 1970, Nature, 227, 680-685, and transferred to a nitrocellulose filter according to the method of Towbin et al., 1979, Proc. Natl. Acad. Sci. USA, 76, 4350-4354. The nitrocellulose membrane is then washed and subsequently blocked with a protein solution (e.g. a 1% bovine serum albumine (BSA), 100mM phosphate buffer, pH 7.4 solution) in order to saturate the nitrocellulose membrane with proteins and to avoid non specific binding of antibodies and labels. In the next step the membrane is then incubated with a specific antibody that binds to proteins which were electrophoretically separated on the gel and transferred to the membrane. Unreacted primary antibody used for binding to the specific protein(s) is washed off and the bound antibody made visible by addition of a enzyme labeled secondary antibody that reacts with the primary antibody. After washing off of the excess secondary antibody, a substrate such as 4-chloro-1-naphtole added to make the bound secondary antibody visible. This technique allows visualizing and determining the molecular weight of proteins reacting with the primary antibody.

### 1) Periodate treatment

The periodate oxidation was performed by means of the incubation of a purified antigen coated microtiter plate with different concentrations of sodium periodate ranging from 0.625 to 100 mM, in 50 mM sodium acetate buffer pH 4.5, for 30 minutes at 37°C. Following a brief rinse with acetate buffer, the plates were incubated with 75 mM sodium borohydride in PBS for 30 minutes at 30°C. Plates were then washed and assayed for antigen immunoreactivity, as described by Woodward M.P. et al, 1985, J. Immunol. Method, 78, 143-153 and Johansson C. et al, 1991, Tumor Biol., 12, 159-170.
The periodate oxidation of LCA resulted in a reduction of the antibody - antigen binding. In the ELISA assay a 50% reduction of the immunoreactivity was measured, using 5mM or 20 mM sodium periodate for an antigen concentration of 0.38 µg/ml or 1 µg/ml, respectively.
These results show that the epitope recognized by P14B4 contains a carbohydrate part.

### 2) Neuraminidase treatment

Neuraminidase digestion was carried out using type X Clostridium perfrigens neuraminidase (Sigma) with equal amounts of the antigen and enzyme for different time intervals (0-240 min), at 37°C, in 50 mM sodium acetate buffer pH 5.0 containing 2 mM CaCl₂.
Samples were analyzed by the ELISA assay, Western Blot and SDS-PAGE analysis, using the conditions described by Gold D.V. et al, 1988, Tumor Biol., 9, 137-144 and Ishida M. et al., 1989, Tumor Biol., 10, 12-22.
Treatment with neuraminidase resulted in a reduction of the immunoreactivity in the ELISA assay: after 30 minutes of digestion with neuraminidase the residual activity was only 13%. Similar results were obtained in a Western Blot analysis where a reduction of the immunoreactivity of the major band of the antigen was found using different times (30-60-240 min) of treatment.
In SDS-PAGE analysis the disappearance of the major band of the antigen may be observed, while a new band at 80 Kd which did not show any immunoreactivity in Western Blot became visible.
These results show that the epitope recognized by P14B4 contains sialyl groups.

### 3) N-glycanase and O-glycanase treatments

Digestion of the carbohydrate moiety with N- and O-glycanase (Genzyme - Cambridge, MA, USA) was performed incubating 800 µg of purified antigen with 20 U of N-glycanase or 50 mU of O-glycanase for 24 h at 37°C in 20 mM sodium phosphate pH 7. Samples alter treatment were tested for immunoreactivity by ELISA assays, as described by Devine P.L., 1991, Cancer Res., 51, 5826-5836.
N-glycanase treatment of the antigen seemed to have no effect on the epitope recognition by ELISA assay, while O-glycanase treatment caused a decrease of immunoreactivity.
These results show that the epitope recognized by P14B4 contains O-glycans.

### 4) Protease treatments

The purified antigen was digested with trypsin (Sigma), chymotrypsin (Sigma), enzymes which specifically cut Lys and Arg sites, and Phe, Trp and Tyr sites, respectively, or pronase (Sigma), a microbial aspecific protease. Digestion were carried out at 37°C for different time intervals (0 - 8 h) incubating 0.1U - 1U of enzymes for 2,5 µg of purified antigen in 80 mM Tris/HCl pH 8.1 (for trypsin and chymotrypsin) or in 40 mM Tris/HCl pH 8.0 (for pronase). Alter digestion samples were analyzed by the ELISA assay, using the conditions described by Johnson V.G. et al., 1986, Cancer Res., 46, 850-857.
Digestion of the antigen with pronase for two hours completely destroyed immunoreactivity, whereas digestion with trypsin and chymotrypsin decreased about 83 % of the immunoreactivity after two hours and 92% after six hours.
These results show that a part of the protein backbone is involved in the epitope recognized by monoclonal antibody P14B4.
Among all treatments reported above, there is a drastic decrease of immunoreactivity only if the group gal β 1 → 3 gal Nac is blocked with PNA lectin, the sialic acid is cleaved out from the antigen or the protein portion is digested by proteases. These results show that the antigenic determinant recognized by P14B4 is characterized by the involvement of the group gal β 1 → 3 gal Nac, the sialic acid and the protein backbone.

### 5) Lectin binding inhibition assay

For the inhibition immunoassay with lectins, the purified antigen, at different concentrations, was incubated in PBS, overnight at 4°C, with 40-400 µg/ml WGA, ConA and PNA lectins, respectively.
All lectins binding with the antigen were analyzed in the ELISA inhibition assay described under point 6) hereafter only PNA lectin inhibited the antigen-antibody recognition.

### 6) Crossreactivity of P14B4 with other heavily glycosylated tumor markers recognised by different monoclonal antibodies

This test was performed using 96 well microtiter plates coated with LCA previously incubated with MAb P14B4 at 37°C for 1 hour. Another equivalent amount of the antigen was incubated in the same conditions with MAb B72.3 (ATCC No. HB 8108) described by Gold D. V. et al., 1988, Tumor Biol., 9, 137-144, as reacting with a core region structure of O-linked carbohydrates. After washing with PBS, the plate was incubated with P14B4-HRP and the colorometric reaction was developed with the method described before. A complete inhibition was observed when LCA was previously incubated with MAb P14B4, but the presence of MAb B72.3 did not seem to inhibit the immunoreactivity of MAb P14B4.
This assay showed that MAb P14B4 recognizes an epitope that does not show any cross-reactivity with the MAb B72.3 epitope.
The crossreactivity of P14B4 with other mucinous antigens was evaluated with a direct ELISA.
The above test was performed with monoclonal antibodies NS 1116, M 11 and OC 125, b-12 or B 72.3 recognizing mucinic tumor markers CA 19.9 (Koprowski H. et al., 1979, Somatic Cell. Genet., 5, 957-971), CA 125 (Bast R.C. et al., 1981, J.Clin. Invest., 68, 1331-37), MCA (Stähli C. et al.,1985, Experientia, 41, 1377-1381) or TAG-72 (Johnson V.G. et al., 1986, Cancer Res., 46, 850-857), respectively. MAb P14B4 did not show any crossreactivity with other mucinic tumor marker.

### Example 7: Comparative analysis of sera of lung cancer patients having different types of lung cancer, by immunoassays for detecting and quantifying LCA, NSE and CYFRA 21.

### 1) Immunoassay for detecting and quantifying LCA

This assay is a heterosandwich two-step immunoassay, which is run either manually or on COBAS® Core Roche immunochemistry analyzer described by Henne V. and Maurer A, 1993, The International Journal of Biological Markers, 8, 2, 133-137.

A first incubation of 30 minutes at 37 °C under permanent shaking is performed with a 20 µl sample, a polystyrene bead coated with monoclonal antibody P14B4 as described by R.H. Burdon et al. pp 297-328, 15, in "Laboratory Techniques in Biochemistry and Molecular Biology", P. Tijssen, 1991, Publisher Elsevier,New York-USA, and 250 µl of a buffer solution, hereafter referred to as "test buffer", containing 100 mM magnesium chloride, 20 mM Tris-HCl, pH 7.4 and 1.3 mg/l bromophenol blue. Unbound components of the test buffer and the sample are washed off with 3-5 ml of distilled water.

250 µl of a buffer solution, hereafter referred to as "conjugate buffer", containing monoclonal antibody P6F7 conjugated to horseradish peroxidase, 20 mM MES (2-morpholino-ethylsulfonic acid) pH 7.4 and 50 µg/ml of non specific mouse monoclonal antibody are added to the washed solid phase and a second incubation is performed with permanent shaking for 30 minutes at 37 °C. Unbound components of the conjugate buffer are washed off with 3-5 ml of distilled water. Substrate 3,3',5,5'-tetramethylbenzidine is added and the development reaction proceeds for another 15 minutes at 37 °C.

Specifically bound conjugate is then quantified by measuring the absorption at 650 nm. The tumor marker concentration is determined using a calibration curve.

### 2) Clinical study

The above described immunoassay, the ENZYMUN-TEST™ CYFRA 21-1 (available from Boehringer Mannheim, FRG) described by Bodenmüller H. et al., p. 137-138, in "Tumor Associated Antigens...", Klapdor et al., 1992, W. Zuckschwerdt Verlag, München, FRG, and the NSE EIA Roche immunoassay (available from Roche, Basel, Switzerland) described by Ebert W. et al., 1990, Tumordiagn. u. Ther., 11, 60-67, were used to detect and quantity the levels of lung cancer markers LCA, CYFRA 21 and NSE, respectively, in serum samples from four groups of persons:
- 48 patients with inflammatory diseases (group 1),
- 56 patients with Small Cell Lung Cancer (SCLC) (group 2),
- 33 patients with Non Small Cell Lung Cancer (NSCLC) adenocarcinoma (group 3), and
- 53 patients with NSCLC squamous cells carcinoma (group 4).
For each lung cancer marker, the ROC curve which represents the variation of sensitivity versus specificity was plotted, as described by Delong E. R., 1985, Biometrics, 41, 947-958, for the combinations of (group 2, group 3 and group 4), (group 2 and group 3), (group 2 and group 4), and (group 3 and group 4), as well as, for group 2, group 3 and group 4, group 1 being the clinically negative group. The sensitivity values deduced from the ROC curves for the specificity values of 90 % and 95 % are set forth in Table 1 hereafter.
As is shown on this table, a higher sensitivity for these two levels of selectivity is attained for all types and combinations of types of lung cancer when using as a tumor marker LCA rather than CYFRA 21 and for all NSCLC types of lung cancer and combinations of types of lung cancer when using as a tumor marker LCA rather than for NSE.
LCA is therefore a better tumor marker than NSE or CYFRA 21 for monitoring patients having a NSCLC lung cancer of any type and for screening persons suspected of having lung cancer, the type of which is not known.

**Table 1**

| Sensitivities of LCA compared to NSE and CYFRA 21 | | | | | | |
|---|---|---|---|---|---|---|
| | LCA | | NSE | | CYFRA 21 | |
| Specificity level | 90% | 95% | 90% | 95% | 90% | 95% |
| clinically positive group(s) considered | | | | | | |
| group 2 + group 3 + group 4 | 74.5 | 68.5 | 47.2 | 40.3 | 56.5 | 54.6 |
| group 2 + group 3 | 70.6 | 63.0 | 66.0 | 44.0 | 51.2 | 48.2 |
| group 2 + group 4 | 69.6 | 63.0 | 56.6 | 50.0 | 54.9 | 50.5 |
| group 3 + group 4 | 81.7 | 78.8 | 34.6 | 27.9 | 64.4 | 63.5 |
| group 2 | 57.0 | 48.3 | 79.3 | 63.8 | 42.9 | 39.2 |
| group 3 | 80.0 | 75.4 | 55.0 | 27.6 | 58.6 | 57.1 |
| group 4 | 91.2 | 89.0 | 31.5 | 26.0 | 76.5 | 70.6 |
| group 1: 48 patients with inflammatory diseases (clinically negative group) group 2: 56 patients with Small Cell Lung Cancer (SCLC) group 3: 33 patients with Non Small Cell Lung Cancer (NSCLC) adenocarcinoma group 4: 53 patients with NSCLC squamous cells carcinoma | | | | | | |

**Table 2**

| Amino-acid composition of LCA | | | |
|---|---|---|---|
| Amino-acid | mg / vial | nmol / vial | number / LCA molecule* |
| Lys | 2.77 | 21.6 | 197.8 |
| His | 1.15 | 8.4 | 76.8 |
| Arg | 2.64 | 16.9 | 154.8 |
| Trp | n. d. ⁺ | n.d.⁺ | n.d.⁺ |
| Asn/Asp | 4.08 | 35.5 | 325 |
| Thr | 3.36 | 33.2 | 304 |
| Ser | 3.62 | 41.5 | 379.8 |
| Glu/Gln | 5.85 | 45.5 | 416.4 |
| Pro | 2.76 | 28.4 | 260 |
| Gly | 1.90 | 33.3 | 304.8 |
| Ala | 2.01 | 28.2 | 258.2 |
| Val | 3.06 | 30.9 | 282.8 |
| Met | 0.59 | 4.5 | 41.2 |
| Ile | 1.55 | 13.7 | 125.4 |
| Leu | 3.53 | 31.2 | 285.6 |
| Thyr | 2.34 | 14.3 | 131 |
| Phe | 2.13 | 14.5 | 132.8 |
| Cys | ----- | ----- | ----- |

| | | | |
|---|---|---|---|
| * assuming a molecular weight of 400 kDa for LCA | | | |
| ⁺ not detected | | | |

## Claims

1. A tumor marker for lung cancer, which is derived from a lung cancer antigen which is a glycoprotein recognized by a monoclonal antibody selected from the group consisting of monoclonal antibodies P14B4 (DSM ACC2170) and P6F7 (DSM ACC2171).

2. A tumor marker according to claim 1, which is recognized by a monoclonal antibody selected from the group consisting of monoclonal antibodies P14B4 and P6F7.

3. A tumor marker according to claim 2, which is a lung cancer antigen recognized by a monoclonal antibody selected from the group consisting of monoclonal antibodies P14B4 and P6F7.

4. A monoclonal antibody recognizing a lung cancer antigen as defined in claim 1 having an apparent molecular weight between 630 and 690 kDa when determined by gel filtration.

5. A monoclonal antibody according to claim 4, characterized in that it is selected from the group consisting of monoclonal antibodies P14B4 and P6F7.

6. A hybridoma cell which produces a monoclonal antibody according to claim 4 or claim 5.

7. A reagent for diagnosis of lung cancer comprising a monoclonal antibody according to claim 4 or claim 5 and a buffer and/or an inert carrier.

8. A reagent according to claim 7, characterized in that it comprises a buffer containing between 10 and 100 mM of magnesium ions and having a pH between 4.0 and 8.0, preferably between 4.5 and 6.5.

9. An imaging agent for lung cancer comprising a monoclonal antibody according to claim 4 or claim 5 linked to a detectable label.

10. An immunotoxin comprising a monoclonal antibody according to claim 4 or claim 5 conjugated to a toxin molecule.

11. A vaccine comprising a tumor marker according to any of claims 1 to 3, in a pharmaceutically acceptable carrier.

12. A method for preparing a tumor marker according to claim 3, said method comprising the steps of
(a) cultivating a population of cells constitutively expressing the lung cancer antigen under conditions which permit the expression thereof,
(b) isolating the tumor marker from the culture and
(c) purifying the tumor marker isolated in step (b).

13. A method for preparing a tumor marker according to any of claims 1 to 3, said method comprising the steps of
(a) culturing a microorganism transformed with a DNA coding for said tumor marker under conditions favorable for expression,
(b) isolating the tumor marker from the culture and
(c) purifying the tumor marker isolated in step (b).

14. A method of isolation and purification of a tumor marker according to claim 3, said method comprising the steps of desalting, immunochromatography and exclusion chromatography.

15. An immunoassay for diagnosing lung cancer, said immunoassay comprising the steps of exposing a sample derived from a body fluid or a lung tissue from an individual suspected of having lung cancer to an antibody recognizing a tumor marker according to any of claims 1 to 3.

16. An immunoassay according to claim 15, characterized in that the antibody is a monoclonal antibody according to claim 4 or claim 5.

17. A method for detecting lung cancer tumors in situ, said method comprising introducing into a patient suspected of having lung cancer an imaging agent according to claim 9 and detecting the accumulation of said imaging agent.

18. A test kit for diagnosing lung cancer, said kit comprising a reagent according to any of claims 7 and 8 and a label.

19. A kit according to claim 18, characterized in that the reagent contains monoclonal antibody P14B4 and the label is linked to monoclonal antibody P6F7.

20. Use of a monoclonal antibody according to claim 4 or claim 5 for diagnosing lung cancer.

21. Use of a kit according to claim 18 or claim 19 for diagnosing lung cancer.
